# EUROPEAN PATENT APPLICATION

(11) **EP 3 342 400 A1**
(43) Date of publication of application: **04.07.2018**
(21) Application number: 18020001.6
(22) Date of filing: 02.01.2018
(51) Int. Cl.: A61K 9/16, A61K 31/4035, A61K 31/41

(54) **A PHARMACEUTICAL COMPOSITION COMPRISING VALSARTAN AND CHLORTHALIDONE**

(30) Priority: 31.12.2016 TR 201620503; 01.08.2017 TR 201711422
(71) Applicant: Abdi Ibrahim Ilac Sanayi ve Ticaret A.S., 34500 Esenyurt/Istanbul (TR)
(72) Inventor: Melik Bugra, Dogru, Esenyurt - Istanbul (TR); Udaya Kumar, Dude, Esenyurt - Istanbul (TR); Sebnem, Sarisan, Esenyurt - Istanbul (TR); Gul Gonul, Kayar, Esenyurt - Istanbul (TR)

(57) **Abstract**

The present invention relates to a fixed-dose combination comprising valsartan and chlorthalidone or a pharmaceutically acceptable salt thereof and at least one pharmaceutically acceptable excipient, and preparation of this combination thereof.

## Description

### Technical Field of the Invention

The present invention relates to a fixed-dose combination comprising valsartan and chlorthalidone or a pharmaceutically acceptable salt thereof and at least one pharmaceutically acceptable excipient, and preparation of this combination thereof.

### Background of the Invention

Valsartan is a specific angiotensin II receptor antagonist selectively and competitively blocking AT₁-receptors mediating vasopressor and aldosterone-related effects of angiotensin II. As it does not inhibit Angiotensin converting enzyme (ACE), it does not give rise to bradykinin accumulation and therefore theoretically it does not lead to such side effects as coughing and angioedema associated with bradykinin accumulation attributable to ACE inhibitors.

Valsartan is administered by oral route. It reaches peak concentrations in plasma through a rapid absorption from the gastrointestinal tract following 2-4 hours of its administration. Within dose range of 80-320 mg, both AUC value and maximum plasma concentration (Cmax) exhibit a linear increase. However, antihypertensive dose-response curve is not linear, and as doses are increased, smaller decreases non-proportional to these increases are obtained in blood pressure.

Valsartan and its production method were first described in the patent EP0443983 (Ciba-Geigy AG). Its chemical name is N-(1-oxopentyl)-N-((2'-(1H-tetrazol-5-yl)(1,1'-biphenyl)-4-yl)methyl)-L-valine and its chemical structure formula is as shown in formula 1.

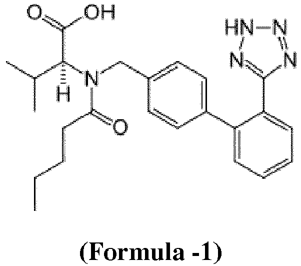

Chlorthalidone is a thiazide-like diuretic used to control hypertension and edema. It is actively used as a long-term monotherapy in decreasing blood pressure, enhancing effects of other hypertensive agents and decreasing cardiovascular events (Ernst, M., et al., N. Engl. J. Med., 361:2153-2164 (2009)). Chemical name of chlorthalidone is 2-chloro-5-(2,3-dihydro-1-hydroxy-3-oxo-1h-isoindole-1-yl)benzenesulfonamide and chemical structure thereof is as shown in the formula 2.

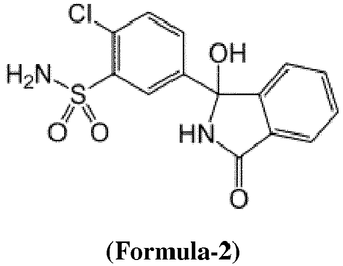

It is similar to thiazides in terms of its structure and pharmacological effects. When it is compared with thiazides, it is the longest acting drug. However, in maximal therapeutic doses, its diuretic effect equals to those of other thiazides. By inhibiting sodium ion transport from the renal tubular epithelium, chlorthalidone increases the excretion of sodium, chlorine and water. It is absorbed from the gastrointestinal tract, but the actual absorbed amount is not known The effect of chlorthalidone starts in 2 hours, reaches its maximum level in 2-6 hours and continues for 48-72 hours.

It is known in the state of the art that the diuretics and angiotensin receptor antagonists can be used alone in treatment of hypertension. However, monotherapy remains insufficient for many patients. In addition, Angiotensin II antagonists can show a synergic effect when used in combination with a diuretic at a lower dose. The combinations available in the market are not sufficient for patients with persistent hypertension. Hence, there is a need for new combinations in treatment of persistent hypertension. The inventors have surprisingly found that the fixed dose combination of valsartan and chlorthalidone provides maximum benefit from the synergistic effect.

### Detailed Description of the Invention

The main objective of the present invention is to produce a fixed-dose combination comprising valsartan and chlorthalidone or a pharmaceutically acceptable salt thereof and at least one pharmaceutically acceptable excipient to be used in treatment of hypertension, diseases associated with hypertension and various cardiovascular diseases.

Another objective of the invention is to produce a stable pharmaceutical composition with high bioavailability and enhanced solubility comprising valsartan and chlorthalidone or a pharmaceutically acceptable salt thereof and at least one pharmaceutically acceptable excipient.

In the present invention, the term "valsartan" refers to valsartan and a pharmaceutically acceptable salt, solvate, polymorph, hydrate or enantiomer thereof or a combination of these.

In the present invention, the term "chlorthalidone" refers to chlortalidone and a pharmaceutically acceptable salt, solvate, polymorph, hydrate or enantiomer thereof or a combination of these.

In the present invention, the term "composition" may also refers to other pharmaceutically acceptable excipients used in the production of pharmaceutical dosage forms. For instance, pharmaceutically acceptable excipients include one or more fillers, disintegrants, lubricants, glidants, colorants, sweeteners and coatings.

Present invention is administered orally. The pharmaceutical composition may be in the form of tablets (film coated tablets, chewable tablets, effervescent tablets, layered tablets, soluble tablets, sublingual tablets, orally disintegrating tablets), capsules (i.e. soft capsules or micro capsules), granule, grain, powder, pills or a combination thereof. Compositions of the present invention are preferably in tablet form and may be optionally coated.

The amount of valsartan in the composition is in the range of 10-480 mg, preferably 80-320 mg, and the amount of chlorthalidone is in the range of 10-30 mg, particularly preferably in the range of 12.5 and 25 mg.

In another embodiment of the invention, invention relates to the pharmaceutical composition comprising 10-480 mg of valsartan and 10-30 mg of chlorthalidone, and production thereof. It was found that a combination of these active substances in these amounts is more effective in reducing high blood pressure to normal levels than valsartan alone, chlorthalidone alone, and currently available valsartan and HCTZ combinations. Furthermore, when chlorthalidone is administered with valsartan, chlortalidone is more effective at the same dose amount than clortalidone monotherapy.

The inventors have encountered some difficulties, especially due to the valsartan compound, when formulating the combination of valsartan and chlorthalidone.

40 mg, 80 mg, 160 mg and 320 mg film tablets comprising valsartan are commercially available in the market. The formulation of these tablets consists of colloidal silicon dioxide, crospovidone, hydroxypropyl methyl cellulose, iron oxides (yellow, black and/or red), magnesium stearate, microcrystalline cellulose, polyethylene glycol 8000 and titanium dioxide. These tablets are prepared by dry granulation method.

Studies showed that stability of these tablets monitored under 40 °C 75% RH conditions, display a decreasing dissolution profile from the 1^{st} month (Table-1).

**Table-1**

| **Commercially Available Reference Product** | **pH 6.8** | | | |
|---|---|---|---|---|
| **Dissolution Medium, 900 ml 50 rpm** | **40 °C 75% RH** | | | |
| | **Initial** | **1^{st} Month** | **3^{rd} Month** | **6^{th} Month** |
| **0 min** | 0.0 | 0.0 | 0.0 | 0.0 |
| **5^{th} min** | 81.7 | 69.0 | 57.0 | 54.0 |
| **10^{th} min** | 93.3 | 88.0 | 77.0 | 75.0 |
| **15^{th} min** | 96.1 | 92.0 | 84.0 | 81.0 |
| **20^{th} min** | 95.8 | 93.9 | 86.0 | 85.0 |
| **30^{th} min** | 95.9 | 93.9 | 86.0 | 85.1 |
| **45^{th} min** | 95.9 | 94.8 | 87.0 | 85.4 |
| **60^{th} min** | 96.4 | 94.5 | 89.2 | 86.2 |

The compositions obtained as a result of the modification to include clortalidone in the present valsartan products exhibit a similar dissolution profile as the existing tablets in the market. However, since the dissolution profile of valsartan decreases from the 1st month in the obtained pharmaceutical composition, the synergistic effect with the combination of chlortalidone decreases and accordingly the therapeutic efficacy decreases. The inventors have surprisingly found that in the formula they developed, dissolution profile of valsartan was improved and there was no significant reduction in dissolution, depending on time.

In another embodiment of the present invention, the inventors determined that wet granulation production method improves bioavailability and dissolution in a fixed dose composition comprising valsartan and chlorthalidone.

Another objective of the present invention is to produce a fixed-dose combination comprising valsartan and chlorthalidone or a pharmaceutically acceptable salt thereof and at least one pharmaceutically acceptable excipient, wherein the composition is produced by wet granulation production method, valsartan fraction is in the intragranular fraction, chlorthalidone fraction is in the extragranular fraction.

Table 2 below shows valsartan dissolution profiles of compositions comprising valsartan in the intragranular phase and chlortalidone in the extragranular phase. Stability of the said composition was monitored at 40 °C 75% RH and analyzed at the end of 1^{st}, 3^{rd}, and 6^{th} months.

**Table-2**

| **Example 1** | **pH 6.8** | | | |
|---|---|---|---|---|
| **Dissolution medium, 900 ml 50 rpm** | **40 °C 75% RH** | | | |
| | **Initial** | **1^{st} Month** | **3^{rd} Month** | **6^{th} Month** |
| **0 min** | 0.0 | 0.0 | 0.0 | 0.0 |
| **5^{th} min** | 86.4 | 85.2 | 78.3 | 80.5 |
| **10^{th} min** | 96.7 | 94.9 | 91.0 | 91.0 |
| **15^{th} min** | 99.3 | 97.0 | 95.2 | 95.3 |
| **20^{th} min** | 100.3 | 97.5 | 96.3 | 97.4 |
| **30^{th} min** | 101.2 | 98.4 | 97.6 | 98.4 |
| **45^{th} min** | 101.6 | 98.5 | 97.9 | 99.3 |
| **60^{th} min** | 102.4 | 98.8 | 99.1 | 99.4 |

The inventors observed that formulation of chlorthalidone active substance in the extragranular phase provided advantages in terms of solubility. However, chlorthalidone active substance formulated in the intragranular phase, does not have the desired dissolution profile in the finished product. From this point of view, the invention also provides an advantage in terms of solubility of the active substance chlorthalidone.

In Table-3, the inventors compared dissolution profiles observed at 30^{th} minute and 60^{th} minute of the two different formulas with respect to formulations of chlorthalidone active substance in intragranular phase and extragranular phase. Results of the comparison are tabulated in Table-4.

**Table-3**

| **Valsartan and Chlorthalidone Film Tablet** | | **Batch No. A** | **Batch No. B** |
|---|---|---|---|
| **S.No** | **Content** | **Yes/No-Tablet** | **Yes/No-Tablet** |
| | **Intragranular** | | |
| 1 | Valsartan | ✔ | ✔ |
| 2 | Lactose | ✔ | ✔ |
| 3 | Colloidal silicon dioxide | ✔ | ✔ |
| 4 | Purified water | ✔ | ✔ |
| 5 | Chlorthalidone | X | ✔ |

| | **Extragranular** | | |
|---|---|---|---|
| 6 | Chlorthalidone | ✔ | X |
| 7 | Lactose | ✔ | ✔ |
| 8 | Low-substituted hydroxypropyl cellulose | ✔ | ✔ |
| 9 | Microcrystalline cellulose | ✔ | ✔ |
| 10 | Colloidal silicon dioxide | ✔ | ✔ |
| 11 | Magnesium stearate | ✔ | ✔ |
| | **Core Tablet** | - | - |
| 12 | **Film Coating** | ✔ | ✔ |
| | **Film Coated tablet** | - | - |

**Table-4**

| **Content** | **Dissolution % at 30^{th} Minute** | | **Dissolution % at 60^{th} Minute** | |
|---|---|---|---|---|
| | **Batch No. A** | **Batch No. B** | **Batch No. A** | **Batch No. B** |
| Valsartan | 98.9 | 96.1 | 100.2 | 97.4 |
| Chlorthalidone | 96.0 | **76.3** | 98.6 | 77.1 |

As can be clearly seen in Table-4, when chlorthalidone active substance is formulated in extragranular phase, it exhibits a high dissolution profile and in intragranular phase exhibits a low dissolution profile.

Another objective of the present invention is that intragranular phase of the composition is formed by formulating valsartan together with at least one pharmaceutical excipient and granulation solvent.

In another embodiment of the present invention, valsartan in intragranular phase is formulated with excipients colloidal silicon dioxide and lactose and a granulation solvent.

The granulation solvent can be water or an organic solvent, or mixtures thereof, but is not limited thereto.

The extragranular phase of the present composition comprises the chlorthalidone active ingredient and at least one pharmaceutically acceptable excipient. Extragranular phase is obtained by mixing intragranular phase with chlorthalidone and at least one pharmaceutically acceptable excipient. Then lubrication process is carried out by using at least one pharmaceutically acceptable lubricant.

In addition, according to BCS classification system, chlorthalidone is classified in Class IV, and therefore it has a low permeability and low solubility. Valsartan, however, is classified in Class II as per BCS classification system, and therefore it has a high permeability and low solubility. Hence, in order to increase the bioavailability of the fixed dose composition comprising valsartan and chlortalidone and to achieve the desired solubility profile, the inventors used chlorthalidone active substance at d50 1 µm to 20 µm, preferably 1 µm to 15 µm, d90 1µm to 40 µm, preferably 1 µm to 35 µm and valsartan active substance at d50 1µm to 20 µm, d90 1µm to 110 µm in the formulation. Particle size can be determined by laser light scattering method via mastersizer 2000.

In the present invention, instead of or in addition to the specifically named excipients mentioned in the above examples, diluents, binders, disintegrants, glidants or lubricants and similar or a mixture thereof may be included.

Fillers/diluents used in invention include, but are not limited to: calcium carbonate, calcium phosphate, dibasic calcium phosphate, tribasic calcium sulfate, calcium carboxymethyl cellulose, cellulose, dextrin derivatives, dextrin, dextrose, fructose, lactitol, Lactose (e.g. Spray dried Lactose, α-Lactose, β-Lactose, Tablettose; in various classes Pharmatose®, Microtose® or Fast-Floc® etc.), (such methylcellulose polymers as Methocel A, Methocel A4C, Methocel A 15C, Methocel A4M; hydroxyethylcellulose, hydroxypropylcellulose, L-hydroxypropylcellulose (low-substituted), hydroxypropyl methylcellulose (HPMC) (for example: Methocel E, F ve K, Shin-Etsu Metolose SH, Methocel F classes, and Metolose 65 SH, Methocel K 4.000, 15.000 and 100.000 cps classes; and Metolose 90 SH 4.000, 15.000, 39.000 and 100.000 classes etc.), sodium carboxymethyl cellulose, carboxymethylene, carboxymethyl hydroxyethyl cellulose, Microcrystalline cellulose and other cellulose derivatives, starches or modified starches (potato starch, wheat starch, corn starch, rice starch, pregelatinized corn starch etc.), magnesium carbonate, magnesium oxide, maltitol, maltodextrins, maltose, glycose, sorbitol, starch, sucrose, sugar and xylitol, erythritol. Preferably, it can comprise at least one of, or a mixture of diluent/filler, lactose, glycose, maltose, sucrose, dextrose, mannitol, maltodextrin, starch, microcrystalline cellulose and calcium phosphate.

Binding agents used in invention include, but are not limited to: crospovidone, polyvinylpyrrolidone (PVP, povidone), polyethylene glycol (PEG), cross-linked polyvinylpyrrolidone, cellulose derivatives (hydroxymethyl cellulose, hydroxypropyl cellulose, methyl cellulose, carboxymethylcellulose sodium, ethyl cellulose, hydroxyethyl cellulose, hydroxypropyl methyl cellulose etc.), sucrose, alginic acid or sodium alginate, carbomer, cottonseed oil, dextrin, dextrose, guar gum, chitosan, stearic acid, hydrogenated vegetable oil type I, magnesium aluminum silica, maltodextrin, maltose, polydextrose, polyethylene oxide, stearic acid and zein. Preferably, binding agent can be at least one of or a mixture of hydroxypropyl cellulose, hydroxypropyl methyl cellulose, hydroxyethyl cellulose, carboxymethyl cellulose, methyl cellulose, chitosan, stearic acid, polyvinylpyrrolidone and crospovidone.

Disintegrants used in invention include, but are not limited to: crospovidone, sodium starch glycolate, hydroxypropyl cellulose, hydroxypropyl methylcellulose, starch, polyvinylpyrrolidone, microcrystalline cellulose, carboxymethyl cellulose sodium or calcium, croscarmellose sodium, pregelatinized starch, polacrilin potassium, sodium or calcium alginate, agar, guar gum, chitosan, alginic acid, sodium alginate and mixtures thereof.

Glidants used in invention include at least one of such substances: colloidal silicon dioxide, talc, aluminum silicate and magnesium silicate or mixtures thereof, but are not limited thereto. The preferred glidant is colloidal silicon dioxide.

An objective of the present invention to produce a pharmaceutical composition comprising valsartan and chlorthalidone or a pharmaceutically acceptable salt thereof and at least one pharmaceutically acceptable excipient, characterized in that the intragranular phase is formed by granulation of valsartan, colloidal silicon dioxide and lactose monohydrate with water; extragranular phase is obtained by mixing chlorthalidone, microcrystalline cellulose, low substituted hydroxypropyl cellulose, lactose and colloidal silicon dioxide with the intragranular phase obtained, and then lubrication is carried out and finally composition is compressed in desired pharmaceutical dosage form. Preferably, it is compressed in tablet form.

The Intragranular phase herein is prepared by mixing of valsartan, colloidal silicon dioxide and lactose in a high shear granulator and granulating with purified water. The obtained granules are dried.

Another objective of the present invention is to produce composition comprising valsartan and chlorthalidone or a pharmaceutically acceptable salt thereof and at least one pharmaceutically acceptable excipient wherein it comprises:
in intragranular phase:
   - in a range of 25-35% valsartan
   - in a range of 0.1-1% colloidal silicon dioxide
   - in a range of 25-35% lactose, and
in extragranular phase:
   - in a range of 1-5% chlorthalidone
   - in a range of 5-20% low-substituted hydroxypropyl cellulose
   - in a range of 5-20% microcrystalline cellulose
   - in a range of 10-30% lactose
   - in a range of 0.1-3% colloidal silicon dioxide
   - in a range of 0.5 -3% magnesium stearate.

The pharmaceutical composition of the invention can be re-formulated by using other pharmaceutically acceptable excipients. In addition, the obtained pharmaceutical composition is designed to be used in treatment of hypertension, diseases associated with hypertension and various cardiovascular diseases.

The present invention will be described more in details by way of the following example. The example does not restrict the scope of the invention and should be considered in the light of the details of the above-given description.

### Example 1:

| **Valsartan - Chlorthalidone (320/25) mg Film Coated tablet** | |
|---|---|
| **Intragranular phase** | |
| Valsartan | 320 mg |
| Colloidal silicon dioxide | 2 mg |
| Lactose | 240 mg |
| Purified water | q.s. |

| **Extragranular phase** | |
|---|---|
| Chlorthalidone | 25 mg |
| Low-substituted hydroxypropyl cellulose | 60 mg |
| Microcrystalline cellulose | 181 mg |
| Lactose | 225 mg |
| Colloidal silicon dioxide | 12 mg |
| Magnesium stearate | 10 mg |

| **Film Coating** | |
|---|---|
| Aquarious Prime BAP314079 Yellow | 25 mg |
| Purified water | q.s. |
| **Total Tablet** | 1100 mg |

Valsartan, colloidal silicon dioxide and lactose are mixed for 10 minutes in the high shear granulator. The mixture is granulated with purified water. The granules are dried in the fluid bed drier and sieved. The obtained granules are mixed with chlorthalidone, microcrystalline cellulose, low-substituted hydroxypropyl cellulose, lactose and colloidal silicon dioxide, and thereafter subjected to lubrication by mixing with magnesium stearate. Following compression, tablets are coated with Aquarious Prime BAP314079 Yellow Film Coating.

## Claims

1. A pharmaceutical composition comprising valsartan and chlorthalidone or a pharmaceutically acceptable salt thereof and at least one pharmaceutically acceptable excipient.

2. A pharmaceutical composition according to Claim 1, wherein amount of valsartan is in the range of 10-480 mg.

3. A pharmaceutical composition according to Claim 1, wherein amount of valsartan is in the range of 80-320 mg.

4. A pharmaceutical composition according to Claim 1, wherein amount of chlorthalidone is in the range of 10-30 mg.

5. A pharmaceutical composition according to Claim 1, wherein amount of chlorthalidone is in the range of 12.5 and 25 mg.

6. A pharmaceutical composition according to Claim 1, wherein it is produced by wet granulation method.

7. A pharmaceutical composition according to Claim 1, wherein valsartan is in intragranular phase and chlorthalidone is in extragranular phase.

8. A pharmaceutical composition according to Claims 1 and 3, wherein intragranular phase is formed by formulating valsartan, at least one excipient and granulation solvent together.

9. A pharmaceutical composition according to Claim 6, wherein granulation solvent is water or organic solvent or mixtures thereof.

10. A pharmaceutical composition according to Claims 1 and 3, wherein extragranular phase includes one or more pharmaceutically acceptable excipients in addition to chlorthalidone.
